# EUROPEAN PATENT APPLICATION

(11) **EP 2 837 390 A1**
(43) Date of publication of application: **18.02.2015**
(21) Application number: 13180567.3
(22) Date of filing: 15.08.2013
(51) Int. Cl.: A61K 45/06, A61K 31/155, A61K 31/198, A61K 31/519, A61K 31/525, A61K 31/661, A61K 31/7076, A61P 21/00

(54) **Combined Pharmaceutical Preparation for Use in Treating Neuromuscular Disorders**

(71) Applicant: Universitäts-Kinderspital beider Basel, 4056 Basel (CH)
(72) Inventor: Fischer, Dirk, 4123 Allschwil (CH)
(74) Representative: Schulz Junghans Patentanwälte PartGmbB

(57) **Abstract**

The present invention relates to a combined pharmaceutical preparation for use in a method for preventing or treating muscular dystrophy, comprising an activator of neuronal nitric oxide synthase, and a nitric oxide precursor, wherein said nitric oxide precursor is convertible by said nitric oxide synthase to nitric oxide

## Description

The present disclosure relates generally to the use of therapeutic agents, preparations and methods for treating muscle diseases and conditions characterized by impaired nitric oxide (NO) signalling in muscle tissue.

There are numerous diseases and conditions that primarily and secondarily affect the muscles and in which an altered NO metabolism is present. Neuromuscular disorders (NMD) are a broad and heterogeneous group of diseases of the peripheral nerve system. NMD include hereditary and acquired disorders of the muscles, the neuromuscular junction, the peripheral nerves, and the second motor neurons within the spinal cord. Muscle diseases, also called myopathies, are primary disorders of skeletal muscle. Hereditary myopathies, which themself represent a large number of different disease entities, include muscle channelopathies, metabolic myopathies, congenital myopathies, and muscular dystrophies. Muscular dystrophy refers to a group of hereditary, progressive, degenerative disorders characterized by progressive degeneration of muscle fibres and muscle tissue over time finally leading to progressive muscle weakness and premature death. Classical histological findings of muscular dystrophies consist of pathological fibre size variation, muscle cell degeneration (necrosis) and regeneration, and replacement of muscle by connective and adipose tissue. Muscular dystrophies include congenital muscular dystrophies (CMD), dystrophinopathies such as Duchenne muscular dystrophy (DMD) and Becker muscular dystrophy (BMD), limb girdle muscular dystrophies (LGMD), distal myopathies, type I and type II myotonic dystrophies (DM1, DM2), facio-scapulo-peroneal muscular dystrophy (FSHD), autosomal and X-linked Emery-Dreifuss muscular dystrophy (EDMD), and oculopharyngeal muscular dystrophy (OPMD). Acquired myopathies include inflammatory myopathies, toxic and drug-induced myopathies, and muscle neoplasm. Disorders of the neuromuscular junction include hereditary (congenital myasthenic syndromes) and acquired immune-positive or antibody negative autoimmune myasthenia gravis. Neurogenic secondary muscle dysfunction and altered NO metabolism is observed in many diseases, such as motor neuron diseases, spinal muscular atrophy (SMA) and amyotrophic lateral sclerosis (ALS), but also in peripheral nerve disorders. Examples of conditions that secondarily affect muscle include muscle atrophy and muscle wasting disorders, including sarcopenia, cachexia, ICU- and surgery-induced weakness, inducible nitric oxide synthase (iNOS) in muscle wasting syndrome, sarcopenia, and cachexia. Sarcopenia is the degenerative loss of skeletal muscle mass and function associated with aging. Cachexia is a multifactorial syndrome resulting in a reduced life expectancy and accompanies many chronic disorders such as cancer, AIDS, sepsis, immune disorders, chronic heart failure, and chronic obstructive pulmonary disease.

Among primary muscle diseases, Duchenne muscular dystrophy (DMD) is the most common inherited muscular dystrophy, affecting almost one in 3500-6000 males, and is caused by mutations in the dystrophin gene. DMD is characterised by progressive muscular weakness affecting proximal muscles more than distal muscles. Onset is usually at five years of age. People with DMD become wheelchair dependent by the time they are 13 years old. All patients are affected by cardiomyopathy by the age of 18. Few survive beyond the third decade; most patients die because of respiratory complications and heart failure due to cardiomyopathy. Becker muscular dystrophy (BMD) is characterised by a later onset and a generally milder clinical course. About one in 20,000 boys is affected by BMD. There are a variety of animal models for DMD, including the mdx mouse, which has served as a standard animal model and which has been used extensively over the years in research.

Deficiency of dystrophin in DMD leads to increased membrane permeability and calcium (Ca²⁺) entry. This results in Ca²⁺ overload in mitochondria directly responsible for increased reactive oxygen species (ROS) generation, which in turn further contributes to membrane damage through peroxidation of sarcolemmal lipids. ROS generation stimulates further Ca²⁺ entry into the muscle fiber, further contributing to mitochondrial dysfunction. Downstream of oxidative stress, ROS also induce activation of NF-κB. A key feature of chronic muscular dystrophies and other neuromuscular disorders is NF-κB activation. NF-kB blockade reduces skeletal muscle degeneration and enhances muscle function in mdx mice. NF-κB induces muscle wasting by stimulating the expression of iNOS, leading to increased nitric oxide (NO) concentrations. Nitric oxide synthases (NOS) are a family of enzymes converting L-arginine to L-citrulline, releasing NO in the process. There are three known NOS isoforms, two are constitutive (neuronal nNOS, endothelial eNOS) isoenzymes, while the third is inducible NOS (iNOS) and involved in inflammatory processes.

iNOS is upregulated in both DMD and mdx muscle samples. NO generated by inducible nitric oxide synthase (iNOS) induces muscle atrophy via regulation of several transcription factors. NO can react with superoxide anions (O²⁻) to form the toxic molecule peroxynitrite (ONOO-), leading to further oxidative stress, and muscle fiber loss. Although the detailed mechanism of how NO-induced stress leads to muscle wasting is yet to be elucidated, the production of NO, and the subsequent formation of peroxynitrite, has been shown to decrease mRNA levels of MyoD - an important transcription factor involved in myogenesis and maintenance of skeletal muscle. On balance, there is strong evidence that stimulation of NO via iNOS contributes to chronic inflammatory changes and muscle atrophy in DMD and other muscular dystrophies. Long term supplementation with L-arginine for 17-months worsened muscle function, increased muscle fibrosis, and increased skeletal deformities in the mdx model suggesting a negative effect on disease progression, which was attributed to iNOS function, suggesting that the NO generated from iNOS is harmful to skeletal muscle (Wehling-Henricks, et al., PLoS One, 2010. 5(5): p. e10763).

nNOS function is commonly impaired in various neuromuscular disorders. A muscle-specific alternatively spliced isoform of nNOS, nNOSµ, is the major source of NO in adult skeletal muscle. In muscle, nNOS localizes to the sarcolemma via direct binding to α-1-syntrophin, a member of the dystrophin-glycoprotein complex (DGC). Not surprisingly, several forms of muscular dystrophy with disruption of the DGC, including DMD and several limb girdle muscular dystrophies (LGMD 2C, 2D, and 2E), display loss of nNOS at the sarcolemma. nNOS activity and NO signalling have different roles in muscle as they modulate blood flow, glucose and fat metabolism, fatigue response, and mitochondrial function. At the level of vasculature, NO offers a prominent mechanism in increasing blood flow to exercising skeletal muscle. With regard to energy metabolism in muscle NO downregulates creatine kinase activity in striated muscle thereby negatively influencing the ATP synthesis and limiting skeletal muscle contractility. Importantly, NO is also capable of influencing oxidative energy metabolism via modulating the mitochondrial respiratory chain complex IV (cytochrome c oxidase / COX). NO competes with molecular oxygen towards binding to the active site of COX, thereby inhibiting its activity. Thus NO reduces oxygen consumption in isolated mitochondria to various extents. In intact cells, physiological levels of NO acutely stimulate uptake and oxidation of glucose and fatty acids by skeletal muscle, while inhibiting the synthesis of glucose, glycogen, and fat. Chronic effects of physiological NO elevation *in vivo* include stimulation of angiogenesis and mitochondrial biogenesis by increasing the concentration SIRT1 and PGC-1α. Genetic enhancement of nNOS expression attenuates skeletal muscle inflammation and necrosis, improves exercise performance, and reduces cardiac fibrosis and contractile dysfunction in the mdx model.

A potential strategy to compensate the impaired nNOS function in DMD consists in supplementation of NO precursors. In the mdx model, a combination of ibuprofen and ISDN (isosorbide dinitrate, a NO precursor) reduced muscle necrotic damage and inflammation and improved free voluntary movement and resistance to exercise. The effects of ISDN and ibuprofen administered separately were transient and significantly lower than those induced by their combination. Furthermore, treatment of mdx mice with a NO donating NSAID (non-steroidal anti-inflammatory drug) also led to muscle function improvement. However, no significant improvement was reported in human DMD patients using a combined approach supplementing a NO donating NSAID (D'Angelo et al., Pharmacol Res, 2012. 65(4): p. 472-9).

A major pathway of nNOS-derived NO is to stimulate cGMP production. Thus, impaired nNOS function may be partially restored with phosphodiesterase 5 (PDE5) inhibitors such as sildenafil. Downstream targets of cGMP include protein kinase G, cyclic nucleotide-gated ion channels, and cGMP-activated PDEs. PDE5 degrades cytosolic cGMP, thereby attenuating NO signaling intensity. Thus, inhibiting PDE5 can raise cytosolic cGMP levels and indirectly amplify NO signaling. PDE5 inhibition in the mdx mice model showed improvement of force in some but not all muscles, reduced fibrosis suggesting positive effects on long term progression but no change of fatigue resistance (Percival et al., J Pathol, 2012. 228(1): p. 77-87).

As previously mentioned, NO is synthesized from L-arginine by nNOS, indicating that L-arginine supplementation could ameliorate DMD. Interestingly, L-citrulline supplementation increases plasma L-arginine concentration and NO-dependent signalling more efficiently than L-arginine supplementation, suggesting that L-citrulline may have a stronger therapeutic effect than L-arginine. Supplementation of NO-precursing substances such as L-arginine has been investigated in animals. Some investigators found improvement of muscle function in the mdx mice. Unfortunately, long term dietary supplementation with L-arginine for 17-months worsened muscle function, increased muscle fibrosis and increased skeletal deformities in the mdx model, suggesting that the application of NO-precursors such as L-arginine or L-citrulline could be harmful to human DMD patients.

Additionally, studies in humans did not show a positive effect of arginine on muscle protein synthesis or muscle function: arginine supplementation neither influences muscle protein synthesis (Tang et al., J. Nutr. 2011, 141, 195-200) nor muscle strengths nor muscle function (Alvares et al., Appl. Physiol. Nutr. Metab. 2012, 37, 115-126).

Furthermore, nNOS is involved in the mechanism of denervation-induced atrophy. nNOS/NO mediates muscle atrophy via regulation of Foxo transcription factors suggesting that nNOS stimulation could be harmful to humans (Suzuki et al., J Clin Invest. 2007 Sep; 117(9):2468-76.).

Thus, the objective of the present invention is to provide means and methods for preventing or treating neuromuscular disorders such as muscular dystrophies, particularly Duchenne muscular dystrophy or Becker muscular dystrophy.

This objective is attained by the subject matter of the independent claims 1, 13, 14, 15 and 16.

Surprisingly it was found that, contrary to the preceding investigations, muscular dystrophies can be treated by combining stimulation of nNOS and supplementation of nitric oxide precursors such as arginine or citrulline.

One aspect of the invention is the provision of a combined pharmaceutical preparation for use in a method for preventing or treating muscular dystrophy, and this preparation comprises an activator of neuronal nitric oxide synthase (CAS Nr 125978-95-2; UniProt P29475) and a nitric oxide precursor, and the nitric oxide precursor is convertible by the nitric oxide synthase to nitric oxide.

Such muscular dystrophy may be selected from congenital muscular dystrophies (CMD), dystrophinopathies such as Duchenne muscular dystrophy (DMD) or Becker muscular dystrophy (BMD), limb girdle muscular dystrophies (LGMD), distal myopathies, type I and type II myotonic dystrophies (DM1, DM2), facio-scapulo-peroneal muscular dystrophy (FSHD), autosomal and X-linked Emery-Dreifuss muscular dystrophy (EDMD), or oculopharyngeal muscular dystrophy (OPMD).

According to the invention the combined pharmaceutical preparation can be administered alone, in which case the active ingredients of the preparation may be administered staggered (at an interval) or in combination, or in combination with one or more other therapeutic agents. Possible combination therapies can take the form of fixed combinations of the combined pharmaceutical preparation of the invention with one or more other therapeutic agents known in the prevention or treatment of muscular dystrophy. The administration can be staggered or the combined agents can be given independently of one another or in the form of a fixed combination.

In some embodiments, the activator of the neuronal nitric oxide synthase is selected from an activator of AMPK (AMP-activated protein kinase, CAS Nr 172522-01-9), 5,6,7,8-tetrahydrobiopterin (BH4, CAS Nr 62989-33-7 and 69056-38-8), FAD (CAS Nr 146-14-5), FMP (CAS Nr 146-17-8), riboflavin (CAS Nr 83-88-5) and NADPH (CAS Nr 53-59-3).

In some embodiments, the nitric oxide precursor is L-arginine and/or L-citrulline.

In some embodiments, the activator of AMPK is selected from:
- N,N-dimethylimidodicarbonimidic diamide (metformin, CAS Nr 657-24-9 or 1115-70-4);
- *N*¹,*N*¹-dimethyl-S-cyclohexyl-*N*⁴-thiohydroxybiguanidie, 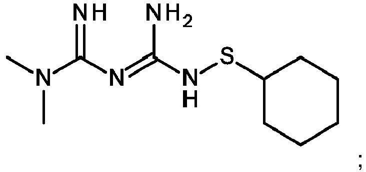
- *N*¹,*N*¹-dimethyl-S-phenyl-*N*⁴-thiohydroxylbiguandine: 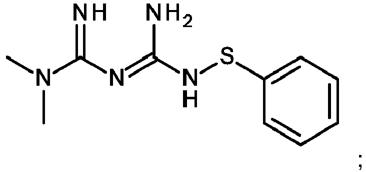
- tert-butyl 4-[(3-(*N,N*-dimethylcabamimidoyl)guanidine)methyl]phenyl carbamate: 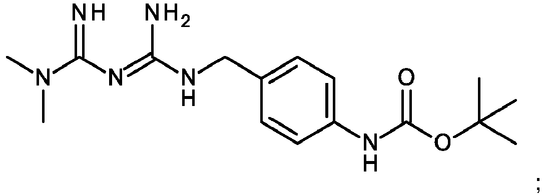
- 4-{[3-(*N,N*-dimethylcabamimidoyl)guanidine]methyl}phenyl octanoate: 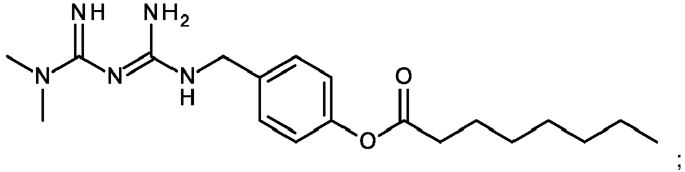
- 4-{[3-(*N,N*-dimethylcabamimidoyl)guanidine]methyl}phenyl diethylcarbamate: 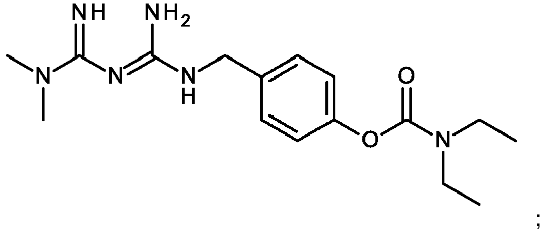
- 4-[(3-(*N*,*N*-dimethylcabamimidoyl)guanidine]methyl]-3-hydroxyphenyl pivalate: 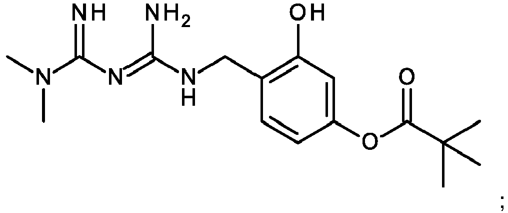
- 3-[3-(*N*,*N*-dimethylcabamimidoyl)guanidine]propyl acetate: 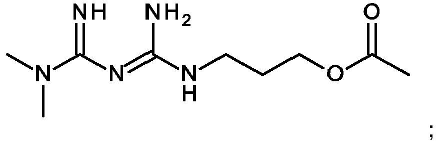
- [(N',N'-dimethylguanidino)iminomethyl]carbamic acid benzyl ester: 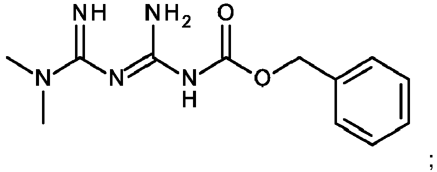
- [(N',N'-dimethylguanidino)iminomethyl]carbamic acid 2,2,2-trichloethyl ester: 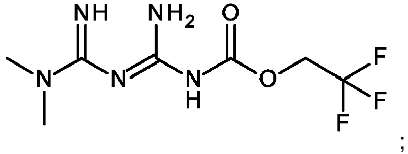 and
- [(*N*',*N*'-dimethylcarbamimidoyl)guanidino]-4-phenyl-1,3,2-dioxaphosphoramidate:

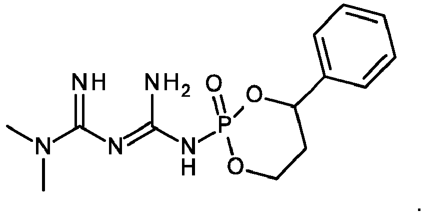

In some embodiments, the combined pharmaceutical preparation comprises metformin and a NO precursor selected from L-arginine and L-citrulline in a mass ratio of 1:5 (metformin: NO precursor), 1:10 (metformin: NO precursor), 1:15 (metformin: NO precursor) or 1:20 (metformin: NO precursor).

In some embodiments, the combined pharmaceutical preparation according to the invention is provided for use in a method for preventing or treating Duchenne muscular dystrophy or Becker muscular dystrophy.

In some embodiments the activator of neuronal nitric oxide synthase and the nitric oxide precursor are present in the same form of administration.

In some embodiments the combined pharmaceutical preparation of the invention is formulated for enteral administration, such as nasal, buccal, rectal (suppository), or oral administration, or as a transdermal or inhalation formulation. In some embodiments, the NO precursor is administered orally and is provided in pulverized form in a sachet. In some embodiments, the combined pharmaceutical preparation is formulated for parenteral administration, such as intravenous, intrahepatic, subcutaneous or intramuscular injection forms.

For parenteral administration the invention gives preference to the use of solutions of a combined pharmaceutical preparation. Suspensions or dispersions are also considered. Especially preferred for parenteral administration are isotonic aqueous solutions, dispersions or suspensions which, for example, can be made up shortly before use. The combined pharmaceutical preparation may be sterilized.

Transdermal/intraperitoneal and intravenous applications are also considered, for example using a transdermal patch, which allows administration over an extended period of time, e.g. from one to forty weeks.

Oral applications are particularly preferred.

In some embodiments the combined pharmaceutical preparation of the invention, wherein particularly the activator of neuronal nitric oxide synthase and the nitric oxide precursor are present in the same form of administration, comprises 100-3000 mg metformin and 1-10 g L-arginine and/or L-citrulline, particularly 175-2000 mg metformin and 1. 5 -5 g L-arginine and/or L-citrulline, and more specifically 250 mg metformin and 2.5 g L-arginine and/or L-citrulline.

In some embodiments the combined pharmaceutical preparation of the invention is provided for use in children and comprises 150 mg to 350mg metformin and 1-4 g L-arginine and/or L-citrulline, particularly 175-300 mg metformin and 1. 5 -3 g L-arginine and/or L-citrulline, and more specifically 250 mg metformin and 2.5 g L-arginine and/or L-citrulline.

In some embodiments the combined pharmaceutical preparation of the invention is provided for use in adults and comprises 300 mg to 700mg metformin and 2-8 g L-arginine and/or L-citrulline, particularly 400-600 mg metformin and 3 - 6 g L-arginine and/or L-citrulline, and more specifically 500 mg metformin and 5 g L-arginine and/or L-citrulline.

In some embodiments the activator of neuronal nitric oxide synthase is present in a first form of administration, and the nitric oxide precursor is present in a second form.

In some embodiments the combined pharmaceutical preparation of the invention comprises 100-400 mg, particularly 175-325 mg, more specifically 250 mg metformin within the first form, and 1-4 g, particularly 1.75 -3.25 g, more specifically 2.5 g L-arginine and/or L-citrulline within the second form.

In some embodiments the metformin and the nitric oxide precursor are formulated for oral administration.

In some embodiments the combined pharmaceutical preparation includes an acceptable pharmaceutical excipient.

The term "excipient" in the context of the present specification particularly refers to a pharmacologically inactive substance that is formulated with the active ingredients.

In some embodiments, such an excipient is used to bulk up the formulation, to increase the stability of the active pharmaceutical ingredient, to control the release of the active pharmaceutical ingredient or to improve the manufacturing of the active pharmaceutical ingredient. In some embodiments the selected excipient is an adherent, a binder, a filler, a disintegrant, a coating, a flavor, a lubricant, a dye or pigment, a sorbent, a gildant, a preservative, a stabilizer, a wetting agent and/or an emulsifier, a solubilizer, a viscosity-increasing agent, a salt for regulating osmotic pressure, a carrier or a buffer substance. Such excipients are prepared in a manner known *per* se, for example by means of conventional dissolving and lyophilizing processes.

In some embodiments, the combined pharmaceutical preparation of the invention for oral administration comprises fillers such as sugars, for example lactose, saccharose, mannitol or sorbitol, cellulose preparations and/or calcium phosphates, and/or binders such as starches, cellulose derivatives and/or polyvinylpyrrolidone, and/or, if desired, disintegrators, flow conditioners and lubricants, for example stearic acid or salts thereof and/or polyethylene glycol. In some embodiments, the combined pharmaceutical preparation of the invention for oral administration is comprised within a tablet, where tablet cores in particular can be provided with suitable, optionally enteric, coatings, and dyes or pigments may be added to the tablets or tablet coatings, for example for identification purposes or to indicate different doses of active ingredient. In some embodiments, the combined pharmaceutical preparation of the invention for oral administration is comprised within hard capsules consisting of gelatin and/or soft, sealed capsules consisting of gelatin and a plasticizer, such as glycerol or sorbitol. In some embodiments, the capsules contain the active ingredient in the form of granules or dissolved or suspended in suitable liquid excipients, such as in oils.

Another aspect of the invention is a dosage regimen provided for preventing or treating muscular dystrophy, where the dosage regimen comprises one, two or three dosage forms comprising 250 mg metformin and 2.5 g arginine and/or or citrulline, or one, two or three dosage forms comprising 250 mg metformin and one, two or three dosage forms comprising 2.5 g arginine and/or citrulline every day.

Another aspect of the invention relates to a dosing regime for children of less than 12 years of age that comprises two or three daily administrations of the combined pharmaceutical preparation of the invention. In some embodiments, such dosing regime provides for three daily administrations each comprising 150 mg to 350mg metformin and 1-4 g L-arginine and/or L-citrulline, particularly 175-300 mg metformin and 1. 5 -3 g L-arginine and/or L-citrulline, and more specifically 250 mg metformin and 2.5 g L-arginine and/or L-citrulline.

In one embodiment, the dosing regime for children provides for three administrations per day of a combined pharmaceutical preparation of 150 mg to 350mg metformin and 1-4 g L-citrulline, specifically 250 mg metformin and 2.5 g L-citrulline, in one combined oral administration form (metformin and L-citrullin being part of the same form).

Another aspect of the invention relates to a dosing regime for adults that comprises three daily administrations of the combined pharmaceutical preparation of the invention, each comprising 300 mg to 700mg metformin and 2-8 g L-arginine and/or L-citrulline, particularly 400-600 mg metformin and 3 - 6 g L-arginine and/or L-citrulline, and more specifically 500 mg metformin and 5 g L-arginine and/or L-citrulline.

In one embodiment, the dosing regime for adults provides for three administrations per day of a combined pharmaceutical preparation of 300 mg to 700mg metformin and 2-8 g L-citrulline, specifically 500 mg metformin and 5 g L-citrulline, in one combined oral administration form (metformin and L-citrullin being part of the same form).

Another aspect of the invention is a form of packaging (kit-of-parts) comprising 7 to 100, particularly 21, 42, 63 or 84, or 14, 28, 42 or 56 dosing forms (corresponding to one, two, three or four weeks of a thrice-daily or twice daily regime, respectively) providing a combined pharmaceutical preparation in accordance with the invention. In some embodiments, the packaging provides 3, 6, 9, 12, 15, 18 or 21 dosing forms on one blister, thereby facilitating following the compliance of a thrice-daily administration regime. In some embodiments, the packaging provides 2, 4, 6, 8, 10, 12 or 14 dosing forms on one blister, thereby facilitating following the compliance of a twice-daily administration regime.

In one embodiment, said blister comprises a joint administration form of the combined pharmaceutical preparation comprising 150 mg to 350mg metformin and 1-4 g L-citrulline, specifically 250 mg metformin and 2.5 g L-citrulline, for use in children, or 300 mg to 700mg metformin and 2-8 g L-citrulline, specifically 500 mg metformin and 5 g L-citrulline for use in adults.

Another aspect of the invention is the provision of a method for the manufacture of a medicament for use in a method for preventing or treating muscular dystrophy, comprising the use of an activator of neuronal nitric oxide synthase and a nitric oxide precursor in combination in accordance with the invention. Medicaments relating to the invention are manufactured by methods known in the art, especially by conventional mixing, coating, granulating, dissolving or lyophilizing.

A further aspect of the invention is the provision of a method for preventing or treating muscular dystrophy, comprising the administration of a combined pharmaceutical preparation in accordance with the invention. According to the invention such treatment may be for prophylactic or therapeutic purposes.

The invention is further characterized, without limitations, by the following example, from which further features, advantages or embodiments can be derived. The examples do not limit the invention but illustrate it.

### Description of the figures

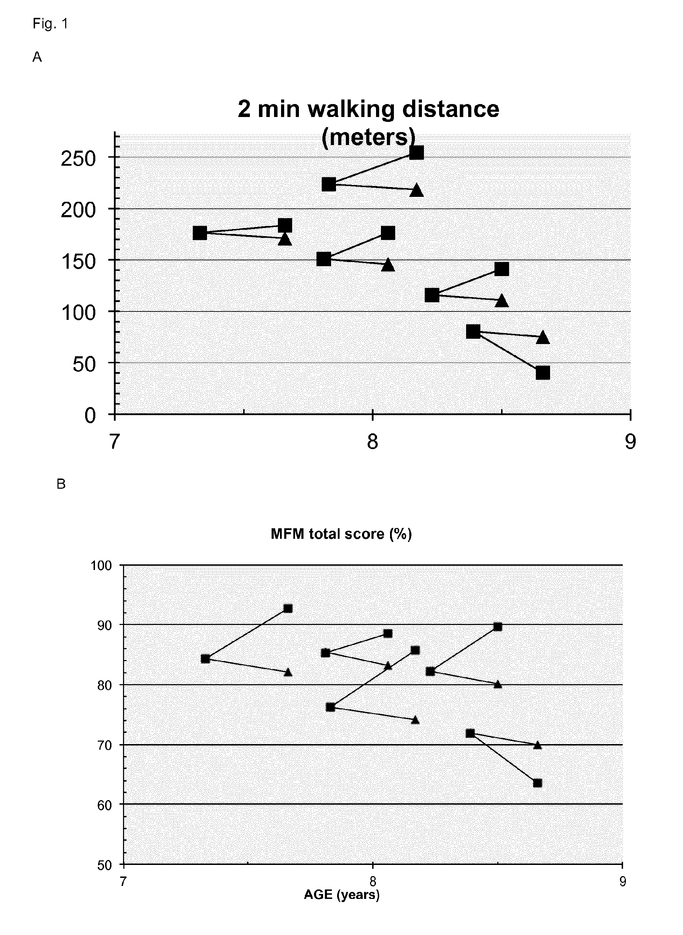

- Fig. 1: shows the individual 2-minute walking distance (2MWD, A) and motor function measure (MFM,B) values at baseline (left point of each set) and after treatment with L-arginine and metformin (squares) and predicted values (triangles) of all DMD patients. The y-axis the 2-minute walking distance in metres (A) and total score in percent of the motor function measure (B), with the X-axis showing the age of the patient (in years) at baseline and after treatment.

### Examples

The present disclosure relates to combinations of therapeutic agents and preparations to treat a skeletal muscle disease or condition characterized by impaired NO-dependent signaling. Among them there are primary muscle diseases such as Duchenne muscular dystrophy (DMD) which is caused by a loss of dystrophin and which is the most common inherited muscular dystrophy. Becker muscular dystrophy (BMD) is the milder variant of dystrophinopathy due to partial dystrophin expression. Embodiments also include other muscular dystrophies, such as congenital muscular dystrophies, limb girdle muscular dystrophies, distal myopathies, type I and type II myotonic dystrophies, facio-scapulo-peroneal muscular dystrophy, autosomal and X-linked Emery-Dreifuss muscular dystrophy, and oculopharyngeal muscular dystrophy. The disease or condition may be treated by administering a combination using a direct or indirect precursor of nitric oxide, such as L-arginine or L-citrulline co-administered with another therapeutic agent that activates the neuronal nitric oxide synthase (nNOS). Also secondary neurogenic muscle dysfunction with impaired NO function as observed in spinal muscular atrophy and amyotrophic lateral sclerosis and finally muscle atrophy and muscle wasting conditions, such as sarcopenia and cachexia, may be treated by this combination.

Exemplary direct nNOS activators are NADPH, flavin adenine dinucleotide, flavin mononucleotide, and tetrahydrobiopterin (BH4), which are cofactors of the nNOS. An indirect AMPK stimulator, and thereby indirect nNOS activator, is metformin. Metformin is known to elevate AMPK intracellular concentrations. Metformin indirectly stimulates AMPK activation by perturbing energy homeostasis. Specifically, it disrupts the oxidative phosphorylation through inhibition of complex I of the electron transport chain, thereby reducing the ATP resynthesis, thereby removing inhibition to AMPK activation. Thus, metformin can be used to stimulate AMPK indirectly, and thereby nNOS.

A combined approach supplementing Duchenne muscular dystrophy patients with L-arginine (NO precursor) and metformin (indirect nNOS activator) for 16 weeks was used. Surprisingly this treatment seems to be superior compared to all other above-mentioned strategies used in DMD patients and in the mdx mice model for DMD, and also superior to the standard treatment of care in DMD (consisting of steroids). Important outcomes in muscular dystrophies and conditions with impaired muscle function are muscle force and function, muscle fatigue resistance, and reducing long term progression (slowing of the progressive replacement of muscle by fat and fibrosis).

No significant improvement had been reported when supplementing a NO releasing anti-inflammatory NSAID in human DMD patients. This approach using NO precursors had been applied to the mdx model with very promising results. In contrast, when assessing muscle force and function using the motor function measure (MFM) scale, patients treated with L-arginine and metformin treated surprisingly showed an important MFM improvement. Also surprisingly, the mean total MFM (+3.5%) and the mean D1 MFM (standing and transfers) subscore (+6%) improved in the L-arginine and metformin treated patients more than the reported MFM and MFM D1 improvement (both improving less than 2%) after beginning the standard (steroid) treatment of care.

In the mdx mice model, supplementation of NO precursing substances such as L-arginine and PDE5 inhibition has been investigated. An improvement of muscle function was observed when supplementing L-arginine and deflazacort. Unfortunately, long term supplementation with L-arginine for 17-months worsened muscle function, increased muscle fibrosis, and increased skeletal deformities in the mdx model suggesting a negative effect on disease progression.

Quantitative muscle magnetic resonance imaging (MRI) showed that DMD patients treated with metformin and L-arginine had a muscle fat content (MFC) increase of quadriceps muscles of only 1.4% corresponding to an annual increase of 4.4%, while we observed that age-matched ambulant DMD patients older than 7 years (group 2 in table 2) receiving standard steroid treatment showed a median annual MFC increase of 10,2%.

This unexpectedly indicates that - in contrast to the observed L-arginine effect in the mdx model - L-arginine and metformin slow the disease progression in human Duchenne patients. As mentioned before, PDE5 inhibition in the mdx model showed improvement of force in some but not all muscles and a reduced fibrosis suggesting positive effects on long term progression, but no change of fatigue resistance. Surprisingly, the approach of the present invention seems also to be superior to PDE5 inhibition. In addition to the observed MFM and quantitative muscle MRI data suggesting improvement of force and slowing of the disease progression, the mean two-minute walking distance of DMD patients treated with L-arginine and metformin also increased (+10 meters), suggesting an improved fatigue resistance as well, an effect not observed with PDE5 inhibition.

### Example 1: Pilot trial with L-arginine and metformin in five ambulant DMD patients

A small pilot study was performed to examine if a 16-week treatment with 3 x 2.5 g/d of L-arginine (NO precursor) and 2 x 250 mg/d metformin (pharmacological AMPK activator) could serve as treatment for DMD. A total of five ambulant DMD patients aged between 7 and 10 years were enrolled and treated in the pilot study. No serious side effects of the study medication were observed. None of the patients dropped out. Laboratory testing revealed no significant changes in creatine kinase levels, markers of renal and liver function, glucose levels, and cholesterol levels (HDL / LDL) comparing baseline vs. post-treatment test results. DEXA scans revealed that the average whole body muscle content (70.6% baseline vs. 70.3% post-treatment) and fat content did not change significantly.

Indirect calorimetry assessing energy and muscle metabolism in vivo showed important changes: the relative carbohydrate contribution to the oxidation rate decreased in all patients (mean change -17.9%), while the relative fatty acid contribution to the oxidation rate increased (mean change 13.9%). Quantitative muscle MRI showed that metformin and L-arginine treated patients had an MFC increase of 1.4% corresponding to an annual increase of 4.4%.

The motor function measure is a validated assessment tool to measure motor function in both ambulant and non-ambulant patients with neuromuscular disorders. It includes items to evaluate three dimensions of motor performance, including specific motor functions, such as transfers and standing posture (D1 subscore). In a recent study on DMD the annual decrease of the total MFM score was 5.8%. In ambulant patients with DMD, D1 was the most informative dimension, with a mean decrease of 17.2% per year before loss of ambulation in patients aged 6 years and over. In contrast, four of the five treated patients showed a marked improvement of their clinical and functional abilities. Surprisingly, the mean total MFM (+3.5%) and the MFM 1 subscore improved by more than +6% in the L-arginine and metformin treated DMD group (Fig. 1 B). Also, the mean two-minute walking distance improved by 9.6 meters indicating improved fatigue resistance (Fig. 1A). Relevant changes observed in the trial are shown in table 1.

**Table 1: Descriptive statistics (mean, median, standard deviation, range, and 95% confidence interval) for selected variables of all five treated patients (change from baseline to post-treatment).**

| **Category** | **Variable** | **mean** | **median** | **SD** | **range** | **95% Cl** |
|---|---|---|---|---|---|---|
| | | | | | | |
| **Calorimetry** | **REE, kcal/24 h** | -51.2 | -56.5 | 37 | -84 to -8 | -54 to -48.5 |
| **Calorimetry** | **carbohydrate oxidation, %** | -17.9 | -11.3 | 18.4 | -44.8 to -4.4 | -20.7 to -15.1 |
| **Calorimetry** | **fatty acid oxidation, %** | 13.9 | 9.6 | 13.7 | 2.9 to 33.6 | 11.1 to 16.7 |
| | | | | | | |
| | | | | | | |
| **Walking** | **2 min. walking distance / meters** | +9.58 | +9.6 | 29.3 | -40.3 to 30.6 | 6.8 to 12.4 |
| | | | | | | |
| | | | | | | |
| **Motor function** | **MFM total score, %** | +3.54 | +7.29 | 6.93 | -8.33 to 8.33 | 0.969 to 6.11 |
| **MFM** | **D1 subscore, %** | +6.15 | +7.69 | 11.6 | -12.8 to 15.4 | 3.58 to 8.72 |
| **MFM** | **D2 subscore, %** | +1.67 | +2.78 | 4.65 | -5.56 to 5.56 | -0.903 to 4.24 |
| **MFM** | **D3 subscore, %** | +1.91 | 0 | 5.43 | -4.76 to 9.53 | -0.665 to 4.48 |

This treatment improves skeletal muscle metabolism and delays muscle degeneration.

## Claims

1. A combined pharmaceutical preparation for use in a method for preventing or treating muscular dystrophy, comprising
- an activator of neuronal nitric oxide synthase, and
- a nitric oxide precursor, which is convertible by said nitric oxide synthase to nitric oxide.

2. The combined pharmaceutical preparation according to claim 1, where said activator is selected from an activator of AMPK, 5,6,7,8-tetrahydrobiopterin, FAD, FMP, riboflavin and NADPH.

3. The combined pharmaceutical preparation according to claim 1 or 2, where said nitric oxide precursor is L-arginine and/or L-citrulline.

4. The combined pharmaceutical preparation according to claim 2 or 3, where said activator of AM PK is selected from
- *N,N*-dimethylimidodicarbonimidic diamide (metformin),
- *N*¹,*N*¹-dimethyl-S-cyclohexyl-*N*⁴-thiohydroxybiguanidie,
- *N*¹,*N*¹-dimethyl-S-phenyl-*N*⁴-thiohydroxylbiguandine,
- tert-butyl 4-[(3-(*N*,*N*-dimethylcabamimidoyl)guanidine)methyl]phenyl carbamate,
- 4-{[3-(*N,N*-dimethylcabamimidoyl)guanidine]methyl}phenyl octanoate,
- 4-{[3-(*N,N*-dimethylcabamimidoyl)guanidine]methyl}phenyl diethylcarbamate,
- 4-[((3-(*N*,*N*-dimethylcabamimidoyl)guanidine]methyl]-3-hydroxyphenyl pivalate,
- 3-[3-(*N*,*N*-dimethylcabamimidoyl)guanidine]propyl acetate,
- [(*N',N*'-dimethylguanidino)iminomethyl]carbamic acid benzyl ester,
- [(*N',N*'-dimethylguanidino)iminomethyl]carbamic acid 2,2,2-trichloethyl ester, and
- [(*N*',*N*'-dimethylcarbamimidoyl)guanidino]-4-phenyl-1,3,2-dioxaphosphoramidate.

5. The combined pharmaceutical preparation according to any one of the preceding claims, wherein said preparation comprises metformin and a NO precursor selected from L-arginine and L-citrulline in a mass ratio of 1:5, 1:10, 1:15 or 1:20.

6. The combined pharmaceutical preparation according to any one of the preceding claims, for use in preventing or treating Duchenne muscular dystrophy or Becker muscular dystrophy.

7. The combined pharmaceutical preparation according to any one of claims 1 to 6, **characterized by** said activator of neuronal nitric oxide synthase and said nitric oxide precursor being present in the same form of administration.

8. The combined pharmaceutical preparation according to claim 7, comprising 100-1000 mg metformin and 1-10 g L-arginine and/or L-citrulline, particularly 250-500 mg metformin and 2,5 - 5 g L-arginine and/or L-citrulline, more particularly specifically 250 mg metformin and 2.5 g L-arginine and/or L-citrulline.

9. The combined pharmaceutical preparation according to any one of claims 1 to 6, **characterized by** said activator of neuronal nitric oxide synthase being present in a first form of administration, and said nitric oxide precursor being present in a second form of administration.

10. The combined pharmaceutical preparation according to claim 9, comprising 100-1000 mg, particularly 250-500 mg, more specifically 250 mg metformin within said first form, and 1-10 g, particularly 2.5 - 5 g, more particularly 2.5 g L-arginine and/or L-citrulline within said second form.

11. The combined pharmaceutical preparation according to any one of claims 5 to 10, **characterized by** metformin and said nitric oxide precursor being formulated for oral administration.

12. A dosage regimen for preventing or treating muscular dystrophy, in particular for preventing or treating Duchenne or Becker muscular dystrophy, comprising one, two or three dosage forms comprising 250 mg metformin and 2.5 g arginine and/or or citrulline, or one, two or three dosage forms comprising 250 mg metformin and one, two or three dosage forms comprising 2.5 g arginine and/or citrulline every day over a period of 10, 15, 16, 20, 30 or 40 weeks.

13. A form of packaging comprising 7 to 31 dosing forms of a combined pharmaceutical preparation according to any one of claims 1 to 12.

14. A method for manufacturing a medicament for preventing or treating muscular dystrophy, comprising the use of a an activator of neuronal nitric oxide synthase and a nitric oxide precursor in combination as defined in one of claims 1 to 13.

15. A method for preventing or treating muscular dystrophy, comprising the administration of a combined pharmaceutical preparation according to any one of claims 1 to 11, or of a dosage regime according to claim 12 to a patient in need thereof.
